# DEMANDE DE BREVET EUROPEEN

(11) **EP 1 097 696 A1**
(43) Date de publication de la demande: **09.05.2001**
(21) Numéro de dépôt: 00402861.9
(22) Date de dépôt: 16.10.2000
(51) Int. Cl.: A61K 7/00, A61K 7/48, A61K 7/06

(54) **Composition contenant des cires et un actif hydrophile instable en milieu oxydant et son utilisation en cosmétique**

(30) Priorité: 08.11.1999 FR 9913993
(71) Demandeur: L'OREAL, 75008 Paris (FR)
(72) Inventeur: Afriat, Isabelle, 75003 Paris (FR)
(74) Mandataire: Rasson, Catherine

(57) **Abrégé**

La présente invention se rapporte à une composition comprenant une phase aqueuse et une phase huileuse comprenant au moins 1% en poids d'une ou plusieurs cires par rapport au poids total de la composition, ladite phase huileuse se présentant, avant le mélange avec une phase aqueuse, sous forme d'une pâte souple à température ambiante et ladite phase aqueuse comprenant au moins un actif hydrophile.

## Description

La présente invention se rapporte à une composition comprenant une phase aqueuse et une phase huileuse comprenant au moins 1 % en poids d'une ou plusieurs cires par rapport au poids total de la composition, ladite phase aqueuse comprenant au moins un actif hydrophile. Elle a trait à l'utilisation de cette composition, plus particulièrement dans le domaine cosmétique, pour la libération de cet actif, notamment en vue de traiter et/ou nettoyer et/ou protéger la peau et/ou les muqueuses et/ou les fibres kératiniques. L'invention se rapporte encore à un procédé de traitement cosmétique qui comprend l'application sur la peau de ladite composition.

La composition de l'invention peut être appliquée, par voie topique, sur le visage, y compris autour des yeux, sur le corps ainsi que sur le cuir chevelu des êtres humains.

Il est connu d'introduire dans les compositions cosmétiques et/ou dermatologiques des actifs en vue d'apporter des traitements spécifiques à la peau et/ou aux cheveux, par exemple pour nettoyer la peau, pour lutter contre le dessèchement, les signes du vieillissement ou la pigmentation de la peau, pour traiter l'acné ou certains désordres de la peau (eczéma, psoriasis), pour combattre les surcharges pondérales, pour favoriser la restructuration de la peau ou son renouvellement cellulaire, pour traiter la séborrhée des cheveux.

Par exemple, on cherche depuis longtemps à formuler l'acide ascorbique (ou vitamine C) dans les domaines cosmétique et dermatologique, sous différentes formes galéniques, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Du fait de ses propriétés, l'acide ascorbique est efficace pour nettoyer la peau et également pour lutter contre les signes du vieillissement de la peau, par exemple pour améliorer l'éclat du teint et estomper les ridules et/ou rides de la peau.

Malheureusement, ces actifs sont souvent instables en milieu oxydant et donc très sensibles à certains paramètres de l'environnement comme par exemple la lumière, l'oxygène et l'eau. Il s'ensuit donc une dégradation rapide de ces actifs lorsqu'ils sont en contact avec notamment un de ces paramètres, ce qui va à l'encontre de l'efficacité recherchée.

Dans l'état de la technique ce problème a été diversement traité. Ainsi, pour diminuer ou retarder la dégradation de l'acide ascorbique en solution, le document US-A-5,140,043 préconise de le stabiliser en l'introduisant dans des solutions hydroalcooliques, formées d'au moins 80 % d'eau et ayant un pH inférieur à 3,5. En raison de la forte acidité de ces solutions, leur utilisation dans le domaine cosmétique est difficilement envisageable. En effet, une application répétée de ces solutions peut perturber l'équilibre de la peau et en particulier irriter, voire brûler la peau.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agroalimentaire). Mais ces techniques sont d'une part coûteuses et peuvent d'autre part altérer l'acide ascorbique, par exemple lors d'un chauffage, et/ou conduire à des compositions peu cosmétiques, comme c'est le cas des granulés.

En outre, le document US-A-5,853,741 décrit la stabilisation de l'acide ascorbique par des silicones élastomères non-émulsifiants connus sous les références commerciales « Gransil » de la société Grand Industrie. L'inconvénient de ces produits est d'apporter un effet huileux, gras, sans effet frais, ce qui ne permet pas ou difficilement leur utilisation en milieu chaud et humide et/ou par les utilisateurs à peaux grasses. De plus ces polymères sont totalement hydrofuges et difficilement incorporables en phase aqueuse. Du fait de leur forte incompatibilité avec l'eau et en particulier avec la sueur, cette dernière n'est pas absorbée par ces polymères et a même tendance à " perler " à la surface de la peau, lorsque celle-ci transpire.

Il subsiste donc le besoin d'une composition utilisable notamment dans le domaine cosmétique, dans laquelle un actif hydrophile et instable en milieu oxydant soit stabilisé, qui soit confortable lors de l'application et qui ne provoque aucune irritation de la peau après application.

La demanderesse a maintenant trouvé une composition surmontant les inconvénients de l'art antérieur.

La présente invention a pour objet une composition comprenant au moins une phase aqueuse et au moins une phase huileuse contenant au moins 1 % en poids d'une ou plusieurs cires par rapport au poids total de la composition, ladite phase huileuse se présentant, avant le mélange avec une phase aqueuse, sous forme d'une pâte souple à température ambiante et ladite phase aqueuse comprenant au moins un actif hydrophile et instable en milieu oxydant.

La présente invention a aussi pour objet l'utilisation d'au moins 1 % en poids d'une ou plusieurs cires dans une composition comprenant au moins une phase huileuse se présentant, avant le mélange avec une phase aqueuse, sous forme d'une pâte souple à température ambiante, et au moins une phase aqueuse comprenant au moins un actif hydrophile qui est instable en milieu oxydant, pour stabiliser le dit actif hydrophile.

On entend par température ambiante une température d'environ 15 à 25°C.

La composition de l'invention bien que contenant un fort taux de cire dans la phase huileuse apporte confort et fraîcheur à l'application sur la peau. Elle se présente sous forme d'une crème, produit souple par opposition à un produit solide tel qu'un stick. On entend par « produit souple » un produit ayant une viscosité allant d'environ 1 à 25 Pa.s, et de préférence de 1 à 10 Pa.s, cette viscosité étant mesurée à la température ambiante (20-25°C) avec un Rhéomat 180.

Selon un mode préféré de réalisation de l'invention, la composition de l'invention a un pH allant de préférence de 5,5 à 7,5.

La composition de l'invention contient, dans la phase huileuse, au moins 1 % et de préférence au moins 5 % en poids d'une ou plusieurs cires, par rapport au poids total de la composition. La phase huileuse se présente, avant le mélange avec une phase aqueuse, sous forme d'une pâte souple. Cette pâte souple est obtenue notamment par chauffage de la cire ou les cires au delà de, ou de préférence jusqu'à, leurs points de fusion et d'éventuels d'autres constituants de la phase huileuse, suivi d'un refroidissement au cours duquel un malaxage est effectué. Ce malaxage est réalisé soit par un broyeur à cylindres, soit par un mélangeur-extrudeur à vis. Cette pâte souple est notamment décrite dans les brevets EP 0667146, EP 0706790, EP 0745376 et EP 0755668. On entend par "pâte souple" une pâte dont on peut mesurer la viscosité, par opposition à la structure solide d'un bâton ou stick, dont on ne peut pas mesurer la viscosité. La viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz.

Comme cires utilisables dans la composition de l'invention, on peut citer par exemple les cires minérales telles que les cires microcristallines, la paraffine, le pétrolatum, la vaseline, l'ozokérite, la cire de montan ; les cires animales telles que la cire d'abeilles, la lanoline et ses dérivés ; les cires végétales telles que les cires de Candellila, d'Ouricurry, de Carnauba, du Japon, le beurre de cacao, les cires de fibres de lièges ou de canne à sucre ; les huiles hydrogénées concrètes à 25°C ; les esters gras et les glycérides concrets à 25°C ; les cires synthétiques telles que les cires de polyéthylène et les cires obtenues par synthèse de Fischer-Tropsch ; les cires de silicone, et leurs mélanges.

Selon un mode préféré de réalisation de l'invention, on utilise au moins une cire ayant une température de fusion commençante supérieure ou égale à 50°C, et mieux au moins une cire dont la température de fusion commençante est supérieure à 65°C, telles que la cire de Carnauba, certaines cires de polyéthylène et certaines cires microcristallines telles que celle vendue par la société Tisco sous le nom "Tisco Wax 88" ou celle vendue par la société RMC sous le nom de "Feruwax 30540".

Par "température de fusion commençante", on entend dans la présente description la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

La quantité de cire(s) dans la composition de l'invention est d'au moins 1 % et va de préférence de 1 à 40 % et mieux de 5 à 20 % en poids par rapport au poids total de la composition.

La phase huileuse de la composition de l'invention comprend généralement, outre la ou les cires, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone tels que les produits commercialisés sous la dénomination "KSG" par la société Shin-Etsu, sous la dénomination "Trefil" par la société Dow Corning ou sous la dénomination "Gransil" par la société General Electric.

De manière avantageuse, la composition de l'invention peut aussi contenir une ou plusieurs charges (constituants pulvérulents) qui peuvent être choisies par exemple dans le groupe formé par le talc ; les micas d'origine naturelle ou synthétique ; le kaolin ; les oxydes de zinc ou de titane ; le carbonate de calcium ; le carbonate et l'hydrocarbonate de magnésium ; la silice, en particulier la silice sphérique, la poudre de silice commercialisée sous la dénomination "Cab-O-Sil TS 530" par la société Cabot, et les microbilles de silice telles que celles commercialisées sous la dénomination SB150 par la société Myoshi ; le dioxyde de titane ; les billes de verre et de céramique commercialisées par la société 3M sous la dénomination commerciale "Macrolite" ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale "Polytrap"), d'acides polyméthacryliques, de polystyrène, de téflon comme le "Fluon" ; les poudres expansées telles que les microsphères creuses en matériau thermoplastique préparées par des procédés connus, comme ceux décrits dans US-A-3 615 972 et EP-A-0 56219 et notamment les microsphères commercialisées sous la dénomination commerciale "Expancel" par la société Kemanord Plast ou sous la dénomination commerciale "Micropearl F 80 ED" par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination "Dry-Flo" par la société National Starch ; les microbilles de résine de silicone telles que celles commercialisées sous la dénomination "Tospearl" par la société Toshiba Silicone, et leurs mélanges.

Les charges peuvent représenter jusqu'à 20 % en poids par rapport au poids total de la composition, et de préférence de 1 à 12 % en poids par rapport au poids total de la composition.

La composition de l'invention permet de stabiliser tout actif hydrophile habituellement instable en milieu oxydant, et plus particulièrement tout actif hydrophile sensible à l'eau, à l'air ou aux métaux. Ainsi, on peut citer, notamment comme actifs hydrophiles instables en milieu oxydant, l'acide ascorbique (vitamine C) et ses dérivés, notamment ses dérivés glycosylés et phosphatés, et ses esters comme l'acétate et le proprionate d'ascorbyle ; l'urée ; l'eau oxygénée ; la procystéïne (l'acide L-2-oxothiazolidine 4-carboxylique), ses sels ou esters (à courtes chaînes : 1 à 6 atomes de carbone) ; la dihydroxyacétone ; la rutine ; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, les dérivés d'acide rétinoique ; l'acide benzène 1,4-di-(3-méthylidène 10-camphosulfonique) ; le glutathion ; les polyphénols ; les dérivés caroténoïdes ; et leurs mélanges.

Il peut s'agir également de tous les composés hydrophiles naturels ou synthétiques pouvant contenir les actifs indiqués ci-dessus, en particulier les extraits végétaux, et plus spécialement les extraits de fruits.

La composition de l'invention est particulièrement intéressante pour stabiliser la vitamine C. Ainsi, la composition selon l'invention conserve de préférence plus de 80%, et avantageusement plus de 90 %, en poids d'acide ascorbique après deux mois à 45°C.

La quantité d'actif hydrophile instable en milieu oxydant dans la composition selon l'invention dépend du type d'actif utilisé et du but recherché. De manière générale, le ou les actifs peuvent être utilisés dans la composition selon l'invention en une quantité allant de 0,01 à 20 % en poids, de préférence de 0,04 à 15 %, et mieux de 0,1 à 10 % en poids par rapport au poids total de la composition.

L'actif hydrophile est introduit dans la phase aqueuse de la composition, notamment par simple mélange.

Ainsi, l'acide ascorbique pulvérulent utilisable dans l'invention peut être celui vendu par Hoffmann-LaRoche; il se présente sous forme de l'acide L-ascorbique avec une pureté de 99% à 100%. Il serait bien entendu possible d'utiliser de l'acide D-ascorbique pulvérulent pur à 99% au moins.

La composition selon l'invention peut donc se présenter sous différentes formes et plus particulièrement sous la forme d'une émulsion, telle qu'une émulsion huile-dans-eau ou eau-dans-huile. On préfère une émulsion eau-dans-huile. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique.

La quantité de la phase huileuse, comprenant au moins 1 % en poids d'une ou plusieurs cires, va généralement de 10 à 75 % en poids par rapport au poids total de la composition selon l'invention. Lorsque la composition est une émulsion, la quantité de cette phase huileuse est comprise de préférence entre 10 et 75 %, avantageusement de 20 à 60 %, en poids par rapport au poids total de la composition. Cette phase huileuse est utilisée en une quantité telle ou bien contient une quantité de cires telle que la quantité de cires dans la composition finale est égale ou supérieure à 1 %.

Lorsque la composition est une émulsion, la quantité de la phase aqueuse est comprise de préférence entre 25 et 90 %, avantageusement de 40 à 80 %, en poids par rapport au poids total de la composition.

Par ailleurs, la composition de l'invention peut être plus ou moins fluide et a notamment l'aspect d'une crème.

Lorsque la composition est une émulsion huile-dans-eau, la composition comprend avantageusement un système émulsionnant. Ce système émulsionnant est avantageusement choisi parmi (1) un mélange d'au moins deux émulsionnants non ioniques, ledit mélange étant liquide à la température ambiante et ayant un HLB allant de 6 à 13, et (2) au moins un émulsionnant anionique liquide à température ambiante.

Par température ambiante, on entend selon l'invention une température allant de 15°C à 25°C.

Le mélange d'émulsionnants non ioniques, liquide à la température ambiante et ayant un HLB allant de 6 à 13, utilisable dans la composition de l'invention, peut comprendre notamment (1) au moins un émulsionnant non ionique ayant un HLB égal ou supérieur à 13 et (2) au moins un émulsionnant non ionique ayant un HLB égal ou inférieur à 5. En outre, il peut comprendre éventuellement au moins un co-émulsionnant qui peut être notamment nécessaire si les émulsionnants (1) et (2) utilisés sont tous deux solides, le co-émulsionnant étant alors liquide et permettant d'obtenir un mélange liquide.

De manière connue, le HLB d'un mélange d'émulsionnants correspond à la moyenne des HLB de chacun des émulsionnants constituant le mélange, compte tenu de la proportion pondérale de ces émulsionnants.

Comme émulsionnants non ioniques de HLB égal ou inférieur à 5, on peut citer par exemple les esters de polyols et d'acide gras ayant une chaîne alkyle comportant de 12 à 22 atomes de carbone, tels que les esters d'acide gras et de glycérine, de glucose ou de sorbitol ; les dérivés oxyéthylénés des esters de polyols et d'acide gras ayant une chaîne alkyle comportant de 12 à 22 atomes de carbone, comportant de 1 à 50 groupes oxyéthylénés, tels que le complexe de triisostéarine (triester de glycérine et d'acide isostéarique) et de PEG-6 ; les éthers de polyéthylène glycol et d'alcool gras ayant une chaîne alkyle comportant de 12 à 22 atomes de carbone, comportant de 1 à 50 groupes oxyéthylénés, tels que les éthers d'oléyle et en particulier l'oleth-25 (25 groupes oxyéthylénés), et leurs mélanges.

Comme émulsionnants non ioniques de HLB égal ou inférieur à 5, on peut utiliser avantageusement ceux qui sont liquides à température ambiante, tels que les esters gras et éthers gras de polyol, à chaîne ramifiée ou insaturée comportant de 12 à 22 atomes de carbone, et en particulier le mono-isostéarate de sorbitan comme le produit vendu sous la dénomination "Arlacel 987" par la société ICI, le mono/di-oléate de sorbitan comme le produit vendu sous la dénomination "Arlacel 83" par la société ICI, le complexe de triisostéarine et de PEG-6 comme le produit vendu sous la dénomination "Labrafil isostearique" par la société Gattefossé, le penta-isostéarate de décaglycéryle comme le produit vendu sous la dénomination "Nikkol Decaglyn 5-IS" par la société Nikko Chemical, le dioléate de méthylglucose comme le produit vendu sous la dénomination "Isolan DO" par la société Goldschmidt.

Comme émulsionnants non ioniques de HLB égal ou supérieur à 13, on peut citer par exemple les esters de polyéthylène glycol et d'acide gras ayant une chaîne alkyle comportant de 12 à 22 atomes de carbone, comportant de 5 à 100 et de préférence de 20 à 60 groupes oxyéthylénés, tels que le stéarate de PEG-40 ; les éthers de polyéthylène glycol et d'alcool gras ayant une chaîne alkyle comportant de 12 à 22 atomes de carbone, comportant de 5 à 100 et de préférence de 10 à 30 groupes oxyéthylénés, tels que le ceteareth-25 ou le ceteth-25 ; les esters de sorbitan et d'acide gras ayant une chaîne alkyle comportant de 12 à 22 atomes de carbone, comportant de 0 à 100 et de préférence de 4 à 25 groupes oxyéthylénés, tels que le Polysorbate 20, le Polysorbate 40 et le Polysorbate 60 ; les esters de sucre et d'acide gras ayant une chaîne alkyle comportant de 12 à 22 atomes de carbone, tels que le stéarate de sucrose ; les dérivés de polyéthylène glycol et d'esters de glycérine et d'acide gras ayant une chaîne alkyle comportant de 12 à 22 atomes de carbone, tels que le PEG-8 caprylic/capric glycerides ; les éthers de polyéthylène glycol et des esters de méthyl glucose et d'acide gras ayant une chaîne alkyle comportant de 12 à 22 atomes de carbone, tels que le PEG-20 methyl glucose sesquistéarate ; et leurs mélanges.

Comme émulsionnants non ioniques de HLB égal ou supérieur à 13, on peut utiliser avantageusement ceux qui sont liquides à température ambiante, tels que le Polysorbate 20 comme le produit vendu sous la dénomination "Tween 20" par la société ICI, le Polysorbate 40 comme le produit vendu sous la dénomination "Tween 40" par la société ICI, le PEG-8 caprylic/capric glycerides comme le produit vendu sous la dénomination "Labrasol" par la société Gattefossé, le PEG-20 methyl glucose sesquistéarate comme le produit vendu sous la dénomination "Glucamate SSE 20" par la société Amerchol.

Comme co-émulsionnant, on peut citer par exemple les alcools gras comportant une chaîne ramifiée ou insaturée ayant de 8 à 22 atomes de carbone, tels que l'alcool isostéarique ; les acides gras comportant une chaîne ramifiée ou insaturée ayant de 8 à 22 atomes de carbone, tels que l'acide ricinoléique ; les esters de polyol et d'acide gras ramifié comportant de 8 à 22 atomes de carbone, tels que les esters gras ramifiés de glycéryle ou de propylène glycol comme l'isostéarate de glycéryle ou l'isostéarate de propylène glycol, et leurs mélanges.

Comme mélange d'émulsionnants non ioniques, liquide à température ambiante et ayant un HLB de 6 à 13, utilisable dans la composition selon l'invention, on peut citer par exemple le mélange de stéarate de glycéryle, stéarate de propylène glycol, isostéarate de glycéryle, isostéarate de propylène glycol, oleth-25 et ceteth-25, commercialisé par la société Gattefosse sous la dénomination "Hydrolactol 70" de HLB 10.

La quantité de mélange d'émulsionnants non ioniques, liquide à la température ambiante et ayant un HLB allant de 6 à 13, et éventuellement de co-émulsionnant, dans la composition selon l'invention va généralement de 0,1 à 30 % en poids en matière active et de préférence de 1 à 25 % en poids en matière active par rapport au poids total de la composition. Dans cette quantité d'émulsionnants, la proportion de chaque émulsionnant peut être aisément déterminée par l'homme du métier en vue d'obtenir un mélange adapté aux critères définis ci-dessus. De manière préférée, la quantité d'émulsionnants non ioniques sans tenir compte du co-émulsionnant va de 0,5 à 10 % en poids et de préférence de 0,5 à 5 % en poids par rapport au poids total de la composition.

L'émulsionnant anionique liquide à température ambiante utilisable dans la composition selon l'invention peut être choisi notamment dans le groupe des tensioactifs anioniques à groupement phosphate, liquides à la température ambiante, tels que les mono, di- et/ou tri-esters d'acide phosphorique et d'alcool gras en C12 à C22, c'est-à-dire ayant une chaîne alkyle comportant de 12 à 22 atomes de carbone, et les mono, di- et/ou tri-esters d'acide phosphorique et d'alcool gras éthoxylé, ayant une chaîne grasse comportant de 12 à 22 atomes de carbone et un nombre de motifs oxyéthylénés allant de 1 à 100 et de préférence de 4 à 25.

On peut citer notamment comme émulsionnant anionique liquide à la température ambiante, le phosphate d'oléyle (mélange de mono- et de diester d'acide phosphorique et d'alcool oléique), le phosphate de trioléyle, le phosphate de dilaureth-4 (mélange de diesters d'acide phosphorique et d'éther de polyéthylène glycol et d'alcool laurique, ayant 4 groupes oxyéthylénés), le phosphate de trioleth-8 (mélange de triesters d'acide phosphorique et d'éther de polyéthylène glycol et d'alcool oléique, ayant 8 groupes oxyéthylénés), le phosphate de triceteareth-4 (mélange de triesters d'acide phosphorique et d'éther de polyéthylène glycol et d'alcools cétylique et stéarylique, ayant 4 groupes oxyéthylénés), le mono-ester d'acide phosphorique et d'acides stéarique et isostéarique (nom CTFA : octyldecyl phosphate) vendu sous la dénomination Hostaphat CG 120 par la société Clariant, et leurs mélanges.

Selon un mode préféré de réalisation de l'invention, on utilise, comme émulsionnant anionique liquide à température ambiante, l'octyldecyl phosphate.

La quantité d'émulsionnant anionique liquide à la température ambiante dans la composition selon l'invention va généralement de 0,1 à 10 % en poids en matière active et de préférence de 1 à 5 % en poids en matière active par rapport au poids total de la composition.

On peut éventuellement ajouter à l'émulsionnant anionique liquide à la température ambiante, un co-émulsionnant du moment que le mélange de l'émulsionnant et du co-émulsionnant est liquide à température ambiante.

Ainsi, comme co-émulsionnant, on peut citer par exemple les alcools gras comportant une chaîne ramifiée ou insaturée ayant de 8 à 22 atomes de carbone, tels que l'alcool isostéarique, les acides gras comportant une chaîne ramifiée ou insaturée ayant de 8 à 22 atomes de carbone, tels que l'acide ricinoléique, et leurs mélanges.

Dans cette variante, quand la composition contient un co-émulsionnant, la quantité de co-émulsionnant va généralement de 0,1 à 10 % en poids en matière active et de préférence de 1 à 5 % en poids en matière active par rapport au poids total de la composition.

Lorsque la composition est une émulsion eau-dans-huile, la composition contient avantageusement au moins 25 % en poids de phase aqueuse par rapport au poids total de la composition et un système émulsionnant comprenant au moins un émulsionnant siliconé. Dans cette variante, la phase aqueuse de la composition de l'invention représente au moins 25 % en poids par rapport au poids total de la composition et de préférence de 40 à 70 % en poids par rapport au poids total de la composition.

L'émulsionnant siliconé utilisable dans la composition de l'invention est alors généralement choisi dans le groupe comprenant les polydiméthylsiloxanes oxyéthylénés et/ou oxypropylénés, les alkyl-C₁₀-C₂₂-polydiméthylsiloxanes oxyéthylénés et/ou oxypropylénés ("alkyl-C₁₀-C₂₂" signifiant que la chaîne alkyle comporte de 10 à 22 atomes de carbone), les polydiméthylsiloxanes oxyéthylénés et/ou oxypropylénés à groupements glucosides, et leurs mélanges. Ces polydimethylsiloxanes peuvent être éventuellement réticulés.

L'émulsionnant siliconé peut être introduit tel quel ou en mélange avec une huile de silicone volatile ou non volatile, telle qu'une cyclométhicone (cyclohexasiloxane, cyclopentasiloxane, cyclotétrasiloxane).

On peut citer comme émulsionnant siliconé utilisable dans la composition de l'invention, les mélanges de dimethicone copolyol et de cyclomethicone vendus sous les dénominations "Q2-3225C" et "DC2-5225C" par la société Dow Corning, le produit vendu sous la dénomination "SF-1288" par la Société General Electric, le mélange de Polyglyceryl-4 isostearate/Cetyl dimethicone copolyol/Hexyl laurate vendu sous la dénomination "Abil WE 09" par la société Goldschmidt, le Cetyl dimethicone copolyol vendu sous la dénomination "Abil EM 90" par la société Goldschmidt, le Laurylmethicone copolyol vendu sous la dénomination "Q2-5200" par la société Dow Corning.

La quantité d'émulsionnant siliconé dans la composition selon l'invention va généralement de 0,1 à 10 % en poids en matière active et de préférence de 2 à 5 % en poids en matière active par rapport au poids total de la composition.

La composition selon l'invention présente en outre l'avantage d'être stable.

De façon avantageuse, cette composition peut être utilisée comme composition cosmétique et/ou dermatologique.

La composition de l'invention peut se présenter sous forme d'un produit à appliquer sur les lèvres, les yeux, la peau et/ou les phanères d'êtres humains. Elle contient donc un milieu physiologiquement acceptable, c'est-à-dire un milieu compatible avec toutes les matières kératiniques telles que la peau aussi bien du corps humain que du visage, les ongles, les cheveux, les cils et les sourcils.

Comme indiqué précédemment, les compositions selon l'invention peuvent être utilisées dans différentes applications topiques, notamment cosmétiques et/ou dermatologiques. La composition selon l'invention peut constituer notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

Aussi, l'invention a encore pour objet l'utilisation cosmétique de la composition selon l'invention pour traiter et/ou nettoyer et/ou protéger la peau et/ou les muqueuses et/ou les fibres kératiniques, c'est-à-dire les cheveux et/ou les cils.

L'invention a aussi pour objet l'utilisation de la composition selon l'invention pour la préparation d'une composition destinée à traiter et/ou nettoyer et/ou protéger la peau et/ou les muqueuses et/ou les fibres kératiniques, c'est-à-dire les cheveux et/ou les cils.

L'invention a aussi pour objet un procédé cosmétique pour nettoyer et/ou traiter et/ou protéger la peau, les muqueuses et/ou les fibres kératiniques, caractérisé en ce qu'il comprend l'application sur la peau et/ou les muqueuses et/ou les fibres kératiniques, d'une composition telle que définie ci-dessus.

La composition selon l'invention peut constituer notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles, les conservateurs, les antioxydants, les séquestrants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 15 % du poids total de la composition. Elle peut contenir également des vésicules lipidiques formées de lipides ioniques ou non ioniques.

Ces adjuvants, selon leur nature, peuvent être introduits dans la phase grasse ou dans la phase aqueuse.

Comme actifs hydrophiles, on peut utiliser par exemple, les polyols, les protéines ou les hydrolysats de protéine, les acides aminés, les hydroxy-acides, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles, le rétinol et ses dérivés.

Bien entendu, l'homme du métier veillera à choisir ce ou ces éventuels adjuvants, et/ou leur quantité, de manière telle que les propriétés avantageuses de la composition selon l'invention ne soient pas, ou substantiellement pas, altérées par l'adjonction envisagée.

La composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte ou un mélangeur-extrudeur à vis. On utilise de préférence un mélangeur-extrudeur à vis.

Un autre objet de l'invention est donc un procédé de préparation d'une composition selon l'invention, caractérisé en ce que l'on effectue au moins une étape du procédé à l'aide d'un broyeur à cylindres ou d'un mélangeur-extrudeur à vis, de préférence d'un mélangeur-extrudeur à vis.

Selon un premier mode de réalisation de l'invention, le procédé de préparation comprend les étapes suivantes :
- (1) préparation de la phase huileuse sous forme d'une pâte souple obtenue par :
   (a) chauffage de la cire ou les cires au delà de, ou de préférence jusqu'à, leurs points de fusion et d'éventuels d'autres constituants, tels que les corps gras, de la phase huileuse,
   (b) un refroidissement jusqu'à température ambiante (15°C à 25°C) au cours duquel un malaxage est effectué, éventuellement d'autres constituants (notamment les charges) de la phase huileuse étant introduits lors de cette étape (b), et en malaxant le mélange obtenu tout en le refroidissant jusqu'à température ambiante tandis qu'il est amené à la sortie du mélangeur-extrudeur,
- (2) éventuellement incorporation d'un système émulsionnant dans la pâte souple obtenue en (1), et
- (3) incorporation, sous agitation, de la phase aqueuse comprenant l'actif hydrophile dans la phase huileuse obtenue en (1) (avec l'éventuel système émulsionnant de (2)) ou inversement (c'est à dire : incorporation de la phase huileuse dans la phase aqueuse).

Le malaxage est donc avantageusement réalisé soit par un broyeur à cylindres, soit par un mélangeur-extrudeur à vis.

Dans ce mode de réalisation, les étapes (2) et (3) sont réalisées lorsque l'on désire obtenir une émulsion simple de type huile-dans-eau ou inversement eau-dans-huile, celles-ci sont mises en oeuvre dans un appareil de mélange habituellement utilisé par l'homme du métier, tel qu'un rotor stator.

En outre, dans le procédé décrit ci-dessus, les quantités utilisées sont telles que la composition finale comprenne au moins 5 % de cire en poids.

Comme indiqué plus haut, le mélange des phases huileuse et aqueuse se faisant à température ambiante (ou à froid), l'incorporation de composés thermosensibles ne pose pas de problème.

Selon un mode particulier de réalisation de l'invention, les étapes (2) et (3) ci-dessus sont également réalisées dans le mélangeur-extrudeur à vis ou le broyeur à cylindres utilisé pour l'étape (1). Le système émulsionnant et la phase aqueuse sont alors introduits dans une partie (ou élément) du mélangeur-extrudeur où la température est proche de la température ambiante.

L'utilisation d'un mélangeur-extrudeur permet d'obtenir une pâte de phase huileuse de qualité très constante de façon reproductible. De plus, il est possible, en adaptant la filière de sortie du mélangeur-extrudeur, de conditionner la composition en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les différentes conditions dans lesquelles l'extrusion peut être effectuée sont décrites dans les brevets EP 0667146, EP 0706790, EP 0745376 et EP 0755668.

Les exemples ci-après de compositions selon l'invention sont donnés à titre d'illustration et sans caractère limitatif. Les quantités y sont données en % en poids, sauf mention contraire.

### Exemple 1 : Emulsion E/H

| | |
|---|---|
| Cire microcristalline | 1,44 % |
| Isoparaffine | 5,54 % |
| Conservateurs | 0,3 % |
| Acide citrique | 1,24 % |
| NaOH | 1,83 % |
| Pentasodium ethylenediamine tetramethylene Phosphonate | 0,05 % |
| Silice | 0,66 % |
| Copolymère éthylène/acide acrylique | 0,72 % |
| Poudre de nylon 12 | 5 % |
| Acrylate copolymer | 0,05 % |
| Cyclomethicone/dimethicone copolyol (DC-5225C) | 20 % |
| Polymethylsesquioxane | 0,49 % |
| Dimethicone/vinyl dimethicone crosspolymer and dimethicone (KSG 16 de la société Shin-Etsu) | 3,08 % |
| Glycérol | 23 % |
| Propylène glycol | 6 % |
| Vitamine C | 5 % |
| Eau | qsp 100 % |

La crème obtenue a une viscosité d'environ 8,6 Pa.s. La vitamine C présente une très bonne stabilité dans cette composition.

### Exemple 2 : exemple comparatif : Emulsion E/H classique

| | |
|---|---|
| Huile d'amandes d'abricots | 3 % |
| Conservateurs | 0,3 % |
| Acide citrique | 1,24 % |
| NaOH | 1,83 % |
| Pentasodium ethylenediamine tetramethylene phosphonate | 0,05 % |
| Poudre de nylon 12 | 5 % |
| Cyclomethicone/dimethicone coppolyol | 20 % |
| Phenyl trimethicone | 4 % |
| Dimethicone/vinyl dimethicone crosspolymer and dimethicone (KSG 16 de la société Shin-Etsu) | 5 % |
| Glycérol | 23 % |
| Propylène glycol | 6 % |
| Vitamine C | 5 % |
| Eau | qsp 100 % |

La stabilité de la vitamine C a été mesurée dans ces deux formules données dans les exemples 1 et 2 ci-dessus. Les résultats sont donnés dans le tableau donné ci-dessous.

| Formules | % perte en vitamine C après 1 mois à 45°C | % perte en vitamine C après 2 mois à 45°C |
|---|---|---|
| Exemple 1 | 0 | 0 |
| Exemple 2 | 3 | 8 |

### Exemple 3 : Emulsion E/H

| | |
|---|---|
| Cire microcristalline | 1,44 % |
| Isoparaffine | 5,54 % |
| Conservateurs | 0,3 % |
| Acide citrique | 1,24 % |
| NaOH | 1,8 % |
| Pentasodium ethylenediamine tetramethylene Phosphonate | 0,1 % |
| Silice | 0,66 % |
| PEG-8 | 6,8 % |
| Copolymère éthylène/acide acrylique | 0,72 % |
| Poudre de nylon 12 | 3 % |
| Acrylate copolymer | 0,05 % |
| Cyclomethicone/dimethicone copolyol (DC-5225 C) | 8 % |
| Cyclométhicone | 10 % |
| Polymethylsesquioxane | 0,49 % |
| Dimethicone/vinyl dimethicone crosspolymer and dimethicone (KSG 16 de la société Shin-Etsu) | 3,08 % |
| Glycérol | 2 % |
| Propylène glycol | 8,5 % |
| Tocophérol | 0,5 % |
| Vitamine C | 5 % |
| Eau | qsp 100 % |

La crème obtenue a une viscosité d'environ 5,4 Pa.s. La vitamine C y reste étonnamment stable malgré le fort pourcentage en eau de la composition.

## Revendications

1. Composition, caractérisée en ce qu'elle comprend au moins une phase aqueuse et au moins une phase huileuse contenant au moins 1 % en poids d'une ou plusieurs cires par rapport au poids total de la composition, ladite phase huileuse se présentant, avant le mélange avec une phase aqueuse, sous forme d'une pâte souple à température ambiante et ladite phase aqueuse comprenant au moins un actif hydrophile et instable en milieu oxydant.

2. Composition selon la revendication 1, caractérisée en ce qu'elle comprend au moins une phase huileuse contenant au moins une cire présentant une température de fusion commençante supérieure ou égale à 50°C, et mieux supérieure à 65°C.

3. Composition selon l'une des revendications 1 ou 2, caractérisée en ce que la quantité de cire(s) dans la composition de l'invention va de 1 à 40 % et de préférence de 5 à 20 % en poids par rapport au poids total de la composition.

4. Composition selon l'une quelconque des revendications précédentes, caractérisée en ce qu'elle comprend une ou plusieurs charges.

5. Composition selon l'une quelconque des revendications précédentes, caractérisée en ce que l'actif hydrophile et instable en milieu oxydant est choisi parmi l'acide ascorbique (vitamine C) et ses dérivés, notamment ses dérivés glycosylés et phosphatés, et ses esters comme l'acétate et le proprionate d'ascorbyle ; l'urée ; l'eau oxygénée ; la procystéïne (l'acide L-2-oxothiazolidine 4-carboxylique), ses sels ou esters; la dihydroxyacétone ; la rutine ; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, les dérivés de l'acide rétinoique ; l'acide benzène 1,4-di-(3-méthylidène 10-camphosulfonique) ; le glutathion ; les polyphénols ; les dérivés caroténoïdes ; et leurs mélanges.

6. Composition selon la revendication précédente, caractérisée en ce que l'actif hydrophile et instable en milieu oxydant est choisi parmi l'acide ascorbique (vitamine C) et ses dérivés.

7. Composition selon l'une quelconque des revendications précédentes, caractérisée en ce que la quantité de la phase huileuse, comprenant au moins 1 % en poids d'une ou plusieurs cires, va de 10 à 75 % en poids par rapport au poids total de la composition.

8. Composition selon l'une quelconque des revendications précédentes, caractérisée en ce qu'elle est sous forme d'une émulsion, de préférence une émulsion eau dans huile.

9. Composition selon l'une quelconque des revendications précédentes, caractérisée en ce qu'elle constitue une composition cosmétique ou dermatologique.

10. Procédé cosmétique pour traiter et/ou nettoyer et/ou protéger la peau, les muqueuses et/ou les fibres kératiniques, caractérisé en ce qu'il comprend l'application sur la peau et/ou les muqueuses et/ou les fibres kératiniques, d'une composition selon l'une quelconque des revendications précédentes.

11. Utilisation cosmétique de la composition selon l'une quelconque des revendications 1 à 9, pour traiter et/ou nettoyer et/ou protéger la peau et/ou les muqueuses et/ou les fibres kératiniques.

12. Utilisation de la composition selon l'une quelconque des revendications 1 à 9, pour la préparation d'une composition destinée à traiter et/ou nettoyer et/ou protéger la peau et/ou les muqueuses et/ou les fibres kératiniques.

13. Utilisation d'au moins 1 % en poids d'une ou plusieurs cires dans une composition comprenant au moins une phase huileuse se présentant, avant le mélange avec une phase aqueuse, sous forme d'une pâte souple à température ambiante, et au moins une phase aqueuse comprenant au moins un actif hydrophile qui est instable en milieu oxydant, pour stabiliser le dit actif hydrophile.

14. Procédé de préparation d'une composition selon l'une quelconque des revendications 1 à 9, caractérisé en ce que l'on effectue au moins une étape du procédé à l'aide d'un mélangeur-extrudeur à vis ou un broyeur à cylindres.

15. Procédé selon la revendication 14. caractérisé en ce qu'il comprend les étapes suivantes :
- (1) préparation de la phase huileuse sous forme d'une pâte souple obtenue par :
(a) chauffage de la cire ou les cires au delà de ou jusqu'à leurs points de fusion et d'éventuels d'autres constituants, tels que les corps gras, de la phase huileuse,
(b) introduction du mélange fondu dans le mélangeur-extrudeur à vis ou le broyeur à cylindres et refroidissement jusqu'à température ambiante au cours duquel un malaxage est effectué, éventuellement d'autres constituants de la phase huileuse étant introduits lors de cette étape (b), et en malaxant le mélange obtenu tout en le refroidissant jusqu'à température ambiante tandis qu'il est amené à la sortie du mélangeur-extrudeur ou du broyeur à cylindres,
- (2) éventuellement incorporation d'un système émulsionnant dans la pâte souple obtenue en (1), et
- (3) incorporation, sous agitation, de la phase aqueuse comprenant l'actif hydrophile dans la phase huileuse obtenue en (1) ou incorporation de la phase huileuse dans la phase aqueuse.

16. Procédé selon la revendications 14 ou 15, caractérisé en ce que la phase huileuse se présente sous forme d'une pâte souple ayant une viscosité dynamique à 25°C comprise entre 3 et 35 Pa.s, mesurée avec un viscosimètre rotatif CONTRAVES TV équipé d'un mobile "MS-r4" à la fréquence de 60 Hz.
